# EUROPEAN PATENT APPLICATION

(11) **EP 2 294 982 A1**
(43) Date of publication of application: **16.03.2011**
(21) Application number: 10159594.0
(22) Date of filing: 12.04.2010
(51) Int. Cl.: A61B 8/00, G01N 29/24

(54) **Probe of ultrasonic diagnostic apparatus and method of suppressing vibration thereof**

(30) Priority: 11.09.2009 KR 20090085865
(71) Applicant: Medison Co., Ltd., Gangwon-do 250-875 (KR)
(72) Inventor: Cho, Joo Yeon, Gangwon-do (KR)
(74) Representative: Schmid, Wolfgang

(57) **Abstract**

A probe (100) of an ultrasonic diagnostic apparatus and a method of suppressing vibration thereof are disclosed. The probe (100) includes a transducer (110) moving along a preset locus, a drive unit (120) driving the transducer (110), a detector (130) sensing real-time vibration of the transducer (110) during movement of the transducer (110), and a controller (140) controlling operation of the drive unit (120) depending on a sensed degree of vibration to suppress the real-time vibration of the transducer (110). Factors related to the drive unit (120) and generating vibration of the transducer (110) can be suppressed by changing a drive force transmitted to the transducer (110) depending on a detected degree of real-time vibration of the transducer (110) to suppress vibration of the transducer (110), thereby improving accuracy in ultrasonic diagnosis and providing a more accurate ultrasound image.

## Description

### BACKGROUND OF THE INVENTION

### 1. Field of the Invention

The present invention relates to a probe of an ultrasonic diagnostic apparatus and, more particularly, to a probe of an ultrasonic diagnostic apparatus that generates internal images of a patient body using ultrasound waves, and a method of suppressing vibration thereof.

### 2. Description of the Related Art

Generally, an ultrasonic diagnostic apparatus refers to a non-invasive apparatus that irradiates an ultrasound signal from a surface of a patient body towards a target internal organ beneath the body surface and obtains an image of a monolayer or blood flow in soft tissue from information in the reflected ultrasound signal (ultrasound echo-signal). The ultrasonic diagnostic apparatus has been widely used for diagnosis of the heart, the abdomen, the urinary organs, and in obstetrics and gynecology due to various merits thereof such as small size, low price, real-time image display, and high stability through elimination of radiation exposure, as compared with other image diagnostic systems, such as X-ray diagnostic systems, computerized tomography scanners (CT scanners), magnetic resonance imagers (MRIs), nuclear medicine diagnostic apparatuses, and the like.

The ultrasonic diagnostic apparatus includes a cart-shaped main body for receiving main components thereof, a probe for transmitting and receiving ultrasound signals, a control panel having various switches and keys for inputting commands for manipulation of the apparatus, and a display unit for displaying an image of an ultrasonic diagnosis result.

The probe includes a transducer that converts electrical signals into sound signals or vice versa. The transducer includes an ultrasound wave vibrator assembly composed of a set of ultrasound wave vibrators, which send ultrasound signals to a target to obtain an image of the target using the signals reflected from the target.

In recent years, with development of image processing techniques, an ultrasonic diagnostic apparatus has been developed to display a three-dimensional ultrasound image. In such an ultrasonic diagnostic apparatus, the probe can obtain an image of a three-dimensional region using a transducer which transmits and receives ultrasound signals while moving along a preset locus.

In such a probe, since it is necessary for the transducer to move right and left to obtain a three-dimensional image, the transducer suffers vibration caused by various factors. When vibration is generated in the transducer, an accurate ultrasound image of a target cannot be obtained. Therefore, there is a need to provide a probe apparatus that overcomes such a problem.

It should be noted that the above description is provided for understanding of the background of the invention and is not a description of a conventional technique well-known in the art.

### SUMMARY OF THE INVENTION

The present invention is conceived to solve the problems of the related art as described above, and an aspect of the invention is to provide a probe of an ultrasonic diagnostic apparatus that can suppress vibration of the probe, and a method of suppressing vibration thereof.

In accordance with one aspect of the invention, a probe of an ultrasonic diagnostic apparatus includes: a transducer moving along a preset locus; a drive unit driving the transducer; a detector sensing real-time vibration of the transducer during movement of the transducer; and a controller controlling operation of the drive unit depending on a sensed degree of vibration to suppress the real-time vibration of the transducer.

The detector may be provided to the transducer.

In accordance with another aspect of the invention, a method of suppressing vibration of a probe of an ultrasonic diagnostic apparatus includes: moving a transducer along a preset locus; sensing real-time vibration of the transducer; and suppressing the real-time vibration of the transducer depending on a sensed degree of vibration.

The sensing real-time vibration of the moving transducer may include sensing the real-time vibration of the transducer using a detector provided to the transducer.

The suppressing the real-time vibration may include suppressing the real-time vibration of the transducer by controlling operation of the drive unit depending on the sensed degree of vibration.

According to embodiments, factors related to the drive unit and generating vibration of the transducer can be suppressed by changing a drive force transmitted to the transducer depending on a detected degree of real-time vibration of the transducer to suppress vibration of the transducer, thereby improving accuracy in ultrasonic diagnosis and providing a more accurate ultrasound image.

### BRIEF DESCRIPTION OF THE DRAWINGS

The above and other aspects, features and advantages of the invention will become apparent from the following description in conjunction with the accompanying drawings, in which:
- Fig. 1: is a perspective view of a probe of an ultrasonic diagnostic apparatus in accordance with one embodiment of the present invention;
- Fig. 2: is a block diagram of the probe in accordance with the embodiment of the present invention;
- Fig. 3: is a cross-sectional view of the probe in accordance with the embodiment of the present invention;
- Fig. 4: is a perspective view of a probe of an ultrasonic diagnostic apparatus in accordance with another embodiment of the present invention; and
- Fig. 5: is a flowchart of a method of suppressing vibration of a probe of an ultrasonic diagnostic apparatus in accordance with one embodiment of the present invention.

### DETAILED DESCRIPTION OF THE EMBODIMENT

Exemplary embodiments of the invention will now be described in detail with reference to the accompanying drawings. It should be noted that the drawings are not to precise scale and may be exaggerated in thickness of lines or size of components for descriptive convenience and clarity only. Furthermore, terms used herein are defined by taking functions of the invention into account and can be changed according to the custom or intention of users or operators. Therefore, definition of the terms should be made according to overall disclosures set forth herein.

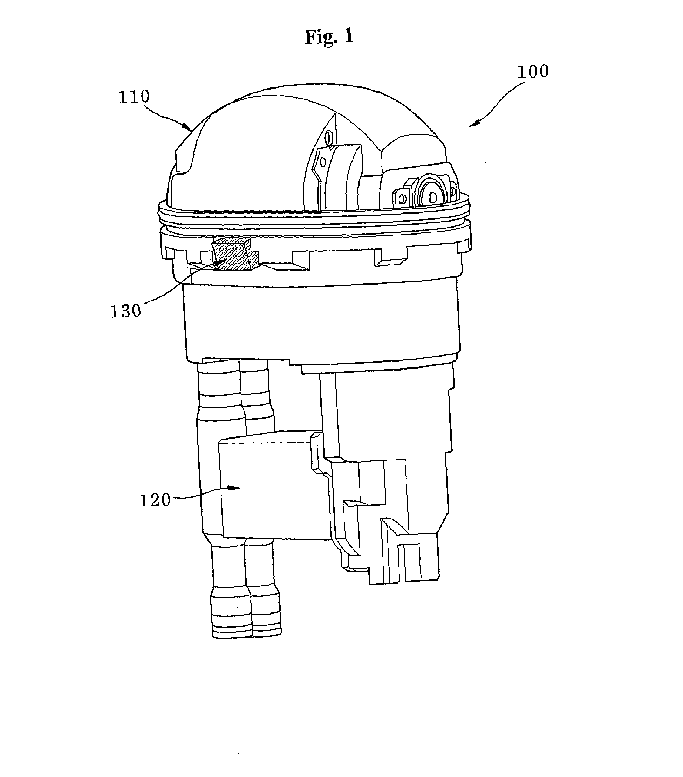

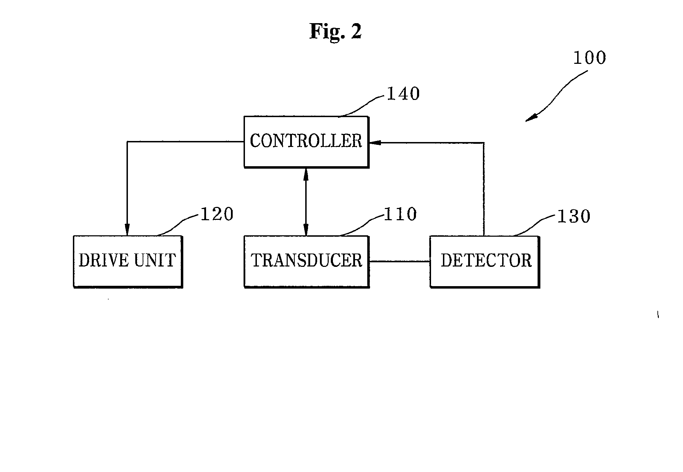

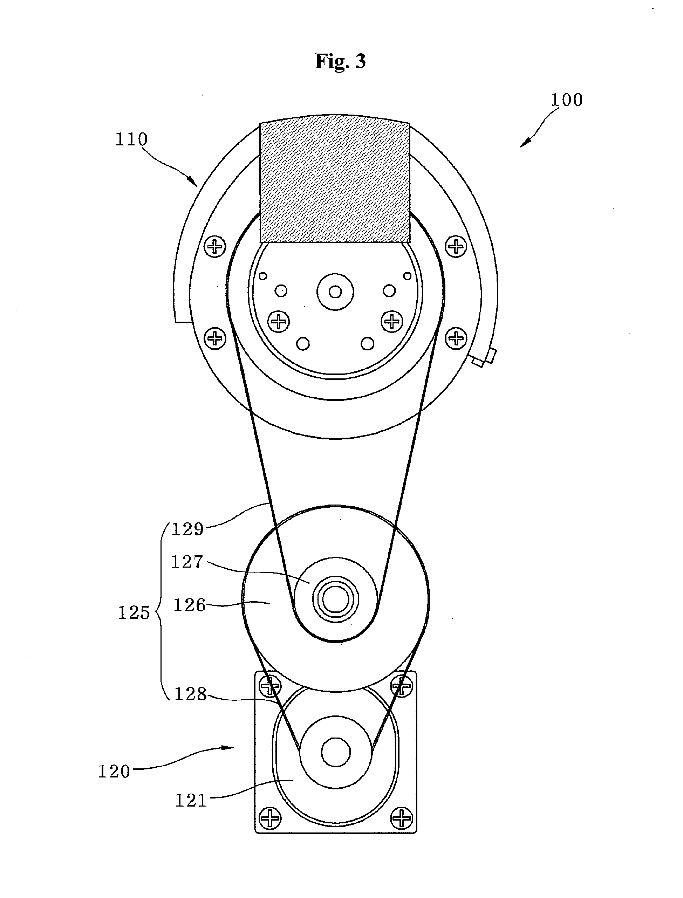

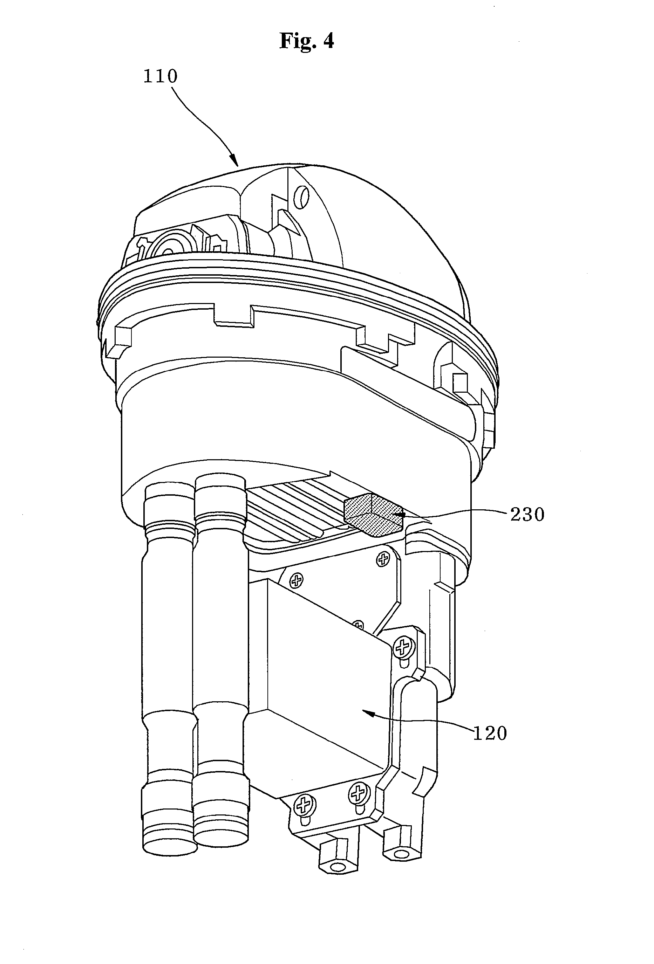

Fig. 1 is a perspective view of a probe of an ultrasonic diagnostic apparatus in accordance with one embodiment of the present invention, Fig. 2 is a block diagram of the probe in accordance with the embodiment of the present invention, Fig. 3 is a cross-sectional view of the probe in accordance with the embodiment of the present invention, and Fig. 4 is a perspective view of a probe of an ultrasonic diagnostic apparatus in accordance with another embodiment of the present invention.

Referring to Figs. 1 to 3, a probe 100 of an ultrasonic diagnostic apparatus according to one embodiment includes a transducer 110, a drive unit 120, a detector 130, and a controller 140.

The transducer 110 moves along a preset locus. The transducer 110 is rotatably disposed inside the probe 100 and transmits an ultrasound signal to a target and receives an ultrasound echo-signal reflected therefrom to realize a three-dimensional image of the target while moving along the preset locus, for example, to be rotated along a predetermined rotational radius.

The transducer 110 includes a piezoelectric layer (not shown) in which a piezoelectric material converts electrical signals into sound signals or vice versa while vibrating, a sound matching layer (not shown) reducing a difference in sound impedance between the piezoelectric layer and a target to allow as much of the ultrasound signals generated from the piezoelectric layer as possible to be transferred to the target, a lens layer (not shown) focusing the ultrasound signals, which travel in front of the piezoelectric layer, onto a predetermined point, and a backing layer (not shown) blocking the ultrasound signals from traveling in the rearward direction of the piezoelectric layer to prevent image distortion.

The drive unit 120 operates the transducer 110. Referring to Fig. 3, the drive unit 120 includes a power generator 121 which generates a drive force, and a power transmission unit 125 which transmits the drive force from the power generator 121 to the transducer 110.

The power transmission unit 125 includes a driving pulley 126 and a driven pulley 127. The driving pulley 126 receives the drive force from the power generator 121 through a driving belt 128 that connects the power generator 121 to the driving pulley 126, and the driven pulley 127 is associated with the driving pulley 126 to transmit the drive force to a driven belt 129 that connects the transducer 110 to the driven pulley 127. When receiving the driving force through the driven pulley 127, the driving belt 129 transmits the driving force to the transducer 110 to allow the transducer 110 to move along a preset locus.

Referring to Figs. 1 and 2, the detector 130 is provided to the transducer 110 or the drive unit 120 and senses real-time vibration of the transducer 110 during movement of the transducer 110. Although, in this embodiment, the detector 130 is provided to the transducer 110, the invention is not limited thereto. Alternatively, the detector 130 may be provided to the drive unit 120 (see Fig. 4) or any other proper position so long as it can sense the vibration of the transducer.

The detector 130 includes a vibration sensor for sensing vibration generated in the probe 100 of the ultrasonic diagnostic apparatus. The detector 130 senses the real-time vibration of the transducer 110 and transmits a detected degree of vibration to the controller 140.

The controller 140 controls operation of the transducer 110 and the drive unit 120. In this embodiment, the controller 140 controls not only fundamental operation of the transducer 110 and the drive unit 120, but also operation of the drive unit 120 in association with the detected degree of vibration to suppress the real-time vibration of the transducer 110. This will be described in detail below.

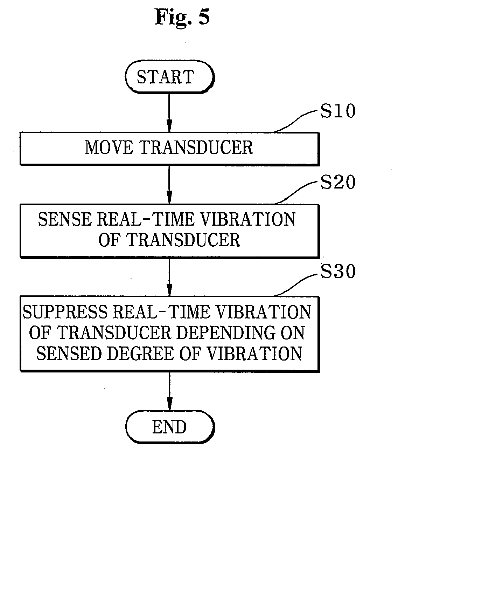

Fig. 5 is a flowchart of a method of suppressing vibration of a probe of an ultrasonic diagnostic apparatus in accordance with one embodiment of the invention.

Referring to Figs. 2 and 5, the method of suppressing vibration of the probe according to this embodiment will be described.

When the probe 100 starts to operate, the power generator 121 of the drive unit 120 is operated to generate a drive force. The drive force generated by the power generator 121 is transmitted to the transducer 110 through the power transmission unit 125, so that the transducer 110 receiving the drive force moves along a preset locus, in S10.

While moving along the preset locus, the transducer 110 can undergo vibration relating to a variety of factors. For example, the transducer 110 can be vibrated by vibration transmitted from the drive unit 120, impact by inertial movement at a point where the transducer 110 changes a moving direction, or other factors such as use conditions, use patterns, or the like.

In this embodiment, the vibration of the transducer 110 is sensed by the detector 130. The detector 130 senses real-time vibration of the transducer 110 and transmits a sensed degree of vibration of the transducer 110 to the controller 140 in real time in S20.

When receiving the sensed degree of vibration of the transducer 110 in real time from the detector 130, the controller suppresses the real-time vibration of the transducer 110 depending on the sensed degree of vibration in S30. In this embodiment, the controller 140 controls operation of the drive unit 120 to change the drive force depending on the sensed degree of vibration, thereby suppressing the real-time vibration of the transducer 110.

For example, the controller 140 compares the degree of vibration, transmitted in real time from the detector 130, with a preset reference degree of vibration, and controls the operation of the drive unit 120 to lower the drive force transmitted from the drive unit 120 to the transducer 110, if the degree of vibration transmitted from the detector 130 exceeds the preset reference degree of vibration.

The controller 140 controls the operation of the drive unit 120 to change the drive force transmitted to the transducer 110 depending on the degree of vibration of the transducer 110 which is sensed in real time, thereby suppressing the vibration of the transducer 110 caused by various factors, such as vibration transmitted from the drive unit 120, impact by movement of the transducer 110 at a point where the transducer 110 changes the moving direction, and the like.

Such controlling the operation of the drive unit 120 is performed to reduce the real-time vibration of the transducer 110 as sensed by the detector 130 to the preset reference degree or less.

In the probe 100 of the ultrasonic diagnostic apparatus according to this embodiment, factors related to the drive unit 120 and generating vibration of the transducer 110 can be suppressed by changing a drive force transmitted to the transducer 110 depending on a detected degree of real-time vibration of the transducer 110 to suppress vibration of the transducer 110, thereby improving accuracy in ultrasonic diagnosis and providing a more accurate ultrasound image.

Although some embodiments have been provided to illustrate the invention in conjunction with the drawings, it will be apparent to those skilled in the art that the embodiments are given by way of illustration only, and that various modifications and equivalent embodiments can be made without departing from the spirit and scope of the invention. Further, the description of the probe as provided herein is only one example of the invention, and the invention can be applied not only to a three-dimensional probe but also to a two-dimensional probe. The scope of the invention should be limited only by the accompanying claims.

## Claims

1. A probe (100) of an ultrasonic diagnostic apparatus, **characterized by** comprising:
a transducer (110) moving along a preset locus;
a drive unit (120) driving the transducer (110);
a detector (130) sensing real-time vibration of the transducer (110) during movement of the transducer (110); and
a controller (140) controlling operation of the drive unit (120) depending on a sensed degree of vibration to suppress the real-time vibration of the transducer (110).

2. The probe (100) according to claim 1, **characterized in that** the detector (130) is provided to the transducer (110).

3. A method of suppressing vibration of a probe of an ultrasonic diagnostic apparatus, **characterized by** comprising:
moving a transducer along a preset locus;
sensing real-time vibration of the transducer; and
suppressing the real-time vibration of the transducer depending on a sensed degree of vibration.

4. The method according to claim 3, **characterized in that** the sensing real-time vibration of the transducer comprises sensing the real-time vibration of the transducer using a detector provided to the transducer.

5. The method according to claim 3 or 4, **characterized in that** the suppressing the real-time vibration comprises suppressing the real-time vibration of the transducer by controlling operation of the drive unit depending on the sensed degree of vibration.
